# EUROPEAN PATENT APPLICATION

(11) **EP 1 589 099 A1**
(43) Date of publication of application: **26.10.2005**
(21) Application number: 03770043.2
(22) Date of filing: 30.10.2003
(51) Int. Cl.: C12N 5/10, C12N 15/09, C12Q 1/02, A61K 35/28, A61K 45/00, A61P 31/12, A61P 35/00

(54) **IMMORTALIZED NATURAL KILLER CELL LINE**

(30) Priority: 30.10.2002 JP 2002316870
(71) Applicant: Japan Science and Technology Agency, Kawaguchi-shi, Saitama 332-0012 (JP)
(72) Inventor: IIZUKA, Satoru, Aoba-ku, Sendai-shi, Miyagi 981-0943 (JP); TAKAI, Toshiyuki, Aoba-ku, Sendai-shi, Miyagi 981-0952 (JP); ITO, Yumi, Shibata-machi, Shibata-gun, Miyagi 989-1 (JP); ITO, Kozue, Aoba-ku, Sendai-shi, Miyagi 981-0933 (JP); OGINATA, Masuo, Aoba-ku, Sendai-shi, Miyagi 980-0871 (JP)
(74) Representative: Williams, Aylsa
(86) International application number: PCT/JP2003/013950
(87) International publication number: WO 2004/039967

(57) **Abstract**

The present invention provides an immortalized natural killer cell line retaining the function and characteristics intrinsic to natural killer cells, a method for establishing the same, a method for screening for useful substances using the immortalized natural killer cell line, and a cell vaccine. By culturing natural killer cells obtained by isolating natural killer cells from the spleen of a transgenic mouse to which a large T-antigen gene of SV40 temperature-sensitive mutant tsA58 is introduced, a cell line which proliferates and activates in the presence of Interleukin-2, has azurophilic granules within cytoplasm, and retains an ability to kill a target cell without presensitization and/or an ability to kill target cells coated with an antibody, is established.

## Description

### Technical Field

The present invention relates to an immortalized natural killer (NK)cell line, in which an immortalized natural killer cell line can be established by subculturing NK cells from spleen cells of a transgenic mouse introduced a large T antigen gene of SV temperature-sensitive mutant tsA58, and its use.

### Background Art

Animals have been conventionally used for the experimental research on pharmaceutical safety and effectiveness. However, from the perspective of animal preservation, technology of experimental research on pharmaceutical effectiveness or safety using cultured cells in vitro has been investigated at a practical level instead of using animals. For example, the experiment of primary culture cells extracted from biogenic tissue, or immortalized cells (established cell) line which proliferate infinitely are carried out prior to the animal experiments. Although primary cells proliferate well in the primary stage, these gradually reduce proliferative ability after the passage of cell culture, and eventually die at the end (this phenomenon is called cell senescence) . Moreover, it has been pointed out that primary cells change their characteristics following the passage, and there is the anxiety that they differ their characteristics every time they are taken from biogenic tissue. Especially, when the primary cells show the slow proliferating speed or these are derived from micro-organ, it is difficult to obtain the primary cells that can be used for tests.

The immortalized cells that acquired infinite proliferation ability, escaped from cell senescence, are accidentally established in the course of repeating the passage of primary culture. However, many of those immortalized cells often lose a part or the whole of morphologically and functionally original characteristics in vivo. Therefore, it has been considered difficult to reflect their intrinsic characteristics of the intact tissue in experiments using such immortalized cell line. With respect to the cell line, attempts to establish immortalized cells with the inherent characteristics of the cells are made by introducing oncogenes such as ras-genes, c-myc genes, Adeno-virus EIA genes, a SV40 virus large T-antigen gene, and human papillomavirus HPV16 genes, maintaining continuously the active proliferating ability of the primary cells. However, even those immortalized cells for some intended organs, since several functions have been already lost at the time when the primary cells were prepared and those oncogenes or large T-antigen genes were introduced, it has been difficult to obtain immortalized cells, in strict terms, maintaining the original function. In particular, it has been extraordinarily difficult to establish cell lines that have a limiting proliferative ability or are derived from microorgans.

A new method has been recently developed for the establishment of immortalized cells by using transgenic technology. These transgenic animals are introduced oncogenes or a large T-antigen gene, which integrated stably into chromosomes, primary cells from organs of the animals already possess oncogenes or a large T-antigen gene at the time of subculturing these, instead of introducing these genes into each cell. In particular, immortalized cells obtained from the organs of a transgenic mouse introduced large T antigen genes of SV40 temperature-sensitive mutant tsA58, are considered to be very effective, because the expression of a SV40 large T gene can be controlled by changing the temperature (Transgenic Research 4, 215-225, 1995; Genes to Cells, 2, 235-244, 1997; Exp. Cell Res. , 197, 50-56, 1991; Exp. Cell Res. , 209, 382-387, 1993; Exp. Cell Res. , 218, 424-429, 1995; Blood, 86, 2590-2597, 1995; Cell. Physiol., 164, 55-64, 1995; Exp. Hematol., 27, 1087-1096, 1999).

On the other hand, natural killer cells are large granular lymphocytes with consisting of 5 to 15% of human peripheral blood lymphocytes, and differentiating from bone marrow, morphologically azurophilic granules within the cytoplasm, they are important cells for maintaining the homeostasis of a living body, in which stressful autologous cells such as tumor cells or virus-infected cells are killed without pre-sensitization of antigens. Moreover, natural killer cells are known to be effective for suppressing proliferation of cancer, more than the killer T cells that are nonspecifically restricted by Major Histocompatibility Complex (MHC) . From such a specific characteristic, natural killer cells are expected to be an advantageous therapy for cancer or virus-induced tumor cells. However, in order to use them for therapy, it is necessary to clarify details of the natural killer cells, including the recognition mechanisms of the target. Furthermore, the establishment of NK cell line has been awaited to make such research easier.

Conventionally, as for a method for proliferating NK cells with high cellular cytotoxicity effectively, the following methods are known: a method for proliferating human natural killer cells with mixed culture of peripheral blood mononuclear leukocyte and human virus tumor cell line HFWT whose proliferation ability is lost previously in the presence of interleukin-2 (Japanese Laid-Open Patent Application No. 2001-149069); an anchorage-dependent cell for stimulation of proliferation in the presence of cells for stimulation of proliferation, wherein a detecting means has been introduced for the purpose of providing human NK cells without contamination of cells for stimulation of proliferation, and a method for proliferating and culturing human NK cells with the use of these cells. (Japanese Laid-Open Patent Application No. 2002-45174). Yet, these methods are aimed for proliferating NK cells under specified conditions to obtain NK cells easily. Despite the establishment of cell line of NK cells, NK cells cannot be, however, generated semipermanently, and they merely disclose methods for proliferating NK cells temporarily.

Although the preparation of NK cell line, which proliferates continuously in a stable manner, is very difficult, four examples as mouse natural killer cell line have been reported so far (Nature, 287, 47-49, 1980; J. Exp. Med. , 181, 1785-1795, 1995; J. Immunol., 158, 112-119, 1997; Int. Immunol., 10, 1093-1101, 1998), while detailed analysis of the NK cells has not been carried out. They haven't merely reported whether these cells have the similar characteristics to the natural killer cells isolated from normal animals. Moreover, as for the cell line in the report on mouse NK cell line, since it is not widely spread and its kinetics is not clear, it is used as materials neither for research and experimentation nor for immune induction, modification, treatment, and the like.

NK cells play an important role in the biogenic defence by recognizing the virus-infected or cancerous cells. Since NK cells kill such transformed cells effectively, administration of NK cells might be useful treatment for infected and cancer cells, while it is necessary to clarify the details of this cell's functions, including the recognition mechanism of the target. In the conventional research of NK cells, NK cells were prepared after disposing mouse, following purification over 2 to 3 hours from spleen and peripheral blood where NK cells reside. This method is disadvantageous and inconvenient, since it spent much time, there was possibly a contamination of cells other than NK cells, and it was necessary to kill mice every time NK cells were prepared. Moreover, life span of prepared NK cells is limited, that is, after one month culture, these cells died even in the presence of cytokine. The object of this invention is to provide an immortalized NK cell line retaining intrinsic characteristics and functions of NK cells, a method for establishing the same, and a method for screening useful substances by using the immortalized NK cell line, or a cell vaccine.

The present inventors made a keen study to solve the above mentioned object. They established an immortalized NK cell line by isolating NK cells from spleen of a transgenic mouse with a large T-antigen gene of SV40temperature-sensitive mutanttsA58, which is immortalized gene, and by subculturing the obtained NK cells for 10 months or more, confirming that the obtained NK cell line showed a stable proliferation, and maintained the characteristics of NK cells. Thus, the present invention has been completed.

### Disclosure of the Invention

Therefore, the present invention is related to an immortalized natural killer cell line derived from spleen cells that proliferates and activates in the presence of Interleukin-2, has azurophilic granules within cytoplasm, and retains an ability to kill a target cell without presensitization and/or an ability to kill a target cells coated with an antibody ("1"); the immortalized natural killer cell line, according to "1," that can proliferate at 33° C, while the proliferation is suppressed at 37°C ("2"); the immortalized natural killer cell line according to "1" or "2," wherein the cell line is derived from a rodent("3"); the immortalized natural killer cell line according to "3", wherein the rodent is a mouse("4"); the immortalized natural killer cell line according to "4," wherein the immortalized natural killer cell line has DX5 , FcγRIII, Ly49H, and CD94 as cell surface antigens, whereas it does not have NK1.1("5") ; the immortalized natural killer cell line according to "4," wherein the immortalized natural killer cell line has FcγRIII and CD94 as cell surface antigens, whereas it does not have NK1.1, DX5 , and Ly49H("6"); the immortalized natural killer cell line according to "4," wherein the immortalized natural killer cell line has NK1.1, Fcγ RIII, and CD94 as cell surface antigens, whereas the immortalized natural killer cell line does not have DX5 and Ly49H("7"); the immortalized natural killer cell line according to "4," wherein the immortalized natural killer cell line has NK1.1, DX5 , FcγRIII and CD94 as cell surface antigens, whereas it does not have Ly49H("8"); the immortalized natural killer cell line according to "4," wherein the immortalized natural killer cell line has FcγRIII, Ly49H, and CD94 as cell surface antigens, whereas it does not have NK1.1 and DX5("9"); and the immortalized natural killer cell line according "4, " wherein the immortalized natural killer cell line has NK1.1, DX5 , FcγRIII, Ly49H, and CD94 as cell surface antigens("10").

The present invention is also related to immortalized natural killer cell line TNK1 (FERM BP-08518)("11"); immortalized natural killer cell line TNK2 (FERM BP-08519) ("12"); immortalized natural killer cell line TNK3 (FERM BP-08520) (Claim 13) ; immortalized natural killer cell line TNK4 (FERM BP-08521)("14"); immortalized natural killer cell line TNK6 (FERM BP-08522)("15"); immortalized natural killer cell line TNK8 (FERM BP-08523)("16"); immortalized natural killer cell line TNK9 (FERM BP-08524)("17"); immortalized natural killer cell line TNK10 (FERM BP-08525) ("18"); and immortalized natural killer cell line TNKb (FERM BP-08526)("19").

Moreover, the present invention is related to a producing method of immortalized natural killer cell line, a method for producing immortalized natural killer cell line, by culturing natural killer cells obtained from spleen of a transgenic mouse, in which a large T-antigen gene of SV40 temperature-sensitive mutant tsA58 is introduced, and by establishing a cell line that proliferates and activates in the presence of interleukin-2, having azurophilic granules within cytoplasm, and retaining an ability to kill a target cell without presensitization, and/or an ability to kill a target cell coated with an antibody ("20"); a method for screening substances promoting or suppressing cellular cytotoxicity of natural killer cells, wherein the immortalized natural killer cells according to any one of "1" to "19" are cultured in the presence of a test substance, and the cellular cytotoxicity level of the immortalized natural killer cells is measured/ evaluated("21"); a substance promoting or suppressing cellular cytotoxicity of the natural killer cells obtained by the screening method according to "21"("22"); a cell having the immortalized natural killer cells according to any one of "1" to "19" as major components ("23"); and the cell vaccine according to "23," wherein the immortalized natural killer cell line can proliferate at 33° C while the proliferation is suppressed at 37°C("24").

### Brief Description of Drawings

Fig. 1 is a set of pictures showing the results of morphologic observation by Wright Giemsa staining method in eight immortalized natural killer cell lines of the present invention.
Fig. 2 is a set of pictures showing results of SV40 detection by PCR, results of SV40T protein detection by Western Blotting method, and temperature-dependent proliferation, in order to confirm that the eight immortalized natural killer cell lines of the present invention are derived from a SV40T transgenic mouse.
Fig. 3 is a figure showing the proliferating factor (Interleukin-2) requirement of the eight immortalized natural killer cell lines of the present invention.
Fig. 4 is a figure showing that the eight immortalized natural killer cell lines of the present invention have cellular cytotoxicity (Natural Killing ability and ADCC ability).
Fig. 5 is a figure showing the analysis result of cell surface antigen of the eight immortalized natural killer cell lines of the present invention.
Fig. 6 is a figure showing that the eight immortalized natural killer cell lines of the present invention have cytokine productive ability.

### Best Mode of Carrying Out the Invention

As for the immortalized NK cell line of the present invention, there is no specific limitation as long as cell line is established from spleen cells that is proliferated/activated by interleukin-2, has azurophilic granules within cytoplasm, and retains an ability (Natural Killing) to kill a target cell without presensitization and/or an ability (ADCC) to kill a target cell coated with an antibody. However, it is preferable that it is a cell line that can proliferate at 33°C , whereas the proliferation is suppressed at 37°C.

As such cell lines, immortalized natural killer cell lines TNK1, TNK4, TNK8 and TNK9 can be exemplified. These immortalized natural killer cell lines are deposited to International Patent Organism Depositary, National Institute of Advanced Industrial Science and Technology, with the deposit numbers FERM BP-08518, FERM BP-08521, FERM BP-08523, FERM BP-08524 (transferred from deposit numbers FERM P-19035, FERM P-19038, FERM P-19040, FERM P-19041 deposited on September 26, 2002), according to Budapest Treaty. Moreover, the origin of the immortalized natural killer cell lines of the present invention is not particularly limited, yet immortalized natural killer cell line derived from rodent such as mice can be preferably exemplified.

As such immortalized natural killer cell lines, immortalized NK cell line which has DX5, FcγRIII, Ly49H and CD94 specific for NK cell surface antigens can be exemplified, whereas it does not have NK1.1. Further, as such immortalized cell line, immortalized natural killer cell line TNK1 (FERM BP-08518) can be exemplified specifically. Furthermore, immortalized NK cell line which has FcγRIII and CD94 can be exemplified as cell surface antigens, whereas it does not have NK1.1, DX5 and Ly49H, and as such immortalized natural killer cell line, immortalized natural killer cell line TNK2 can be exemplified. This immortalized natural killer cell line TNK2 is deposited to International Patent Organism Depositary, National Institute of Advanced Industrial Science and Technology, with the deposit numbers of FERM BP-08519, (transferred from deposit numbers FERM P-19036 deposited on September 26, 2002), according to Budapest Treaty. Furthermore,immortalized natural killer cell line which has NK1. 1, FcγRIII and CD94 as cell surface antigens can be exemplified, whereas it does not have DX5 and Ly49H, and as such immortalized natural killer cell lines, immortalized NK cell lines TNK3, TNK6, TNK10 and TNKb can be specifically exemplified. These immortalized natural killer cell lines are deposited to International Patent Organism Depositary, National Institute of Advanced Industrial Science and Technology, with the deposit numbers FERM BP-08520, FERM BP-08522, FERM BP-08525, FERM BP-08526, (transferred from deposit numbers FERMP-19037, FERMP-19309, FERMP-19042, FERMP-19043 deposited on September 26, 2002), according to Budapest Treaty. Also, the immortalized NK cell line as cell surface antigens which has NK1.1, DX5 , FcγRIII and CD94 can be exemplified, whereas it does not have Ly49H, and as such immortalized NK cell line, immortalized NK cell line TNK4 (FERM BP-08521) can be exemplified. Besides, immortalized natural killer cell line which has FcγRIII, Ly49H, and CD94 as cell surface antigens can be exemplified, whereas it does not have NK1.1 and DX5 , and as such immortalized natural killer cell line, immortalized natural killer cell line TNK8 (FERMBP-08523) can specifically be pointed out. Moreover, immortalized natural killer cell line having NK1.1, DX5 , FcγRIII, Ly49, and CD94 as cell surface antigens, can be exemplified, and as such immortalized natural killer cell line, immortalized natural killer cell line TNK9 (FERM BP-08524) can be specifically pointed out.

As for the method for producing immortalized natural killer cell line of the present invention, it is not particularly limited as long as it is a method for producing immortalized natural killer cell line, by culturing natural killer cells obtained from spleen of a transgenic mouse in which a large T-antigen gene of SV40 temperature-sensitive mutant tsA58 is introduced, and by establishing a cell line that proliferates and activates in the presence of interleukin-2. These cells have azurophilic granules within cytoplasm, and retain an ability to kill a target cell without presensitization and/or a target cell coated with an antibody. For example, it is possible to establish immortalized NK cell line, by isolating NK cells from spleen of the above-mentioned transgenic mouse by NK 1.1 ⁺ MACS (magnetic beads method), following the subculture of the isolated NK cells for 10 months or more in a medium containing 1000U/mL of interleukin-2, a cytokine promoting proliferation, in the presence of 5% of CO₂, at 33° C.

The above transgenic mouse can be also generated as follows. For example, a DNA fragment having tsA58 large T-antigen gene is prepared as follows: plasmid pSVtsA58 (-) - 2 (Ohno T. et al. , Cytotechnology 7, 165-172, 1991) introduced in pBR322 by opening ring of the total DNA of tsA58ori(-)-2 wherein the origin of replication (ori) of SV40 is deleted, with the restriction of enzyme BamHI, is amplified in large amounts in E. coli , and the amplified plasmid is digested with restriction enzyme BamHI to remove the vector part. By introducing this DNA fragment into totipotent cells of animals such as rodents animals including mice, in which a promoter of large T-antigen cells exists, according to a common procedure, congenic mice having a large T-antigen gene of SV40 temperature-sensitive mutant tsA58 into all the cells, that is, transgenic mice, can be generated. In such transgenic mice, a large T-antigen gene of tsA58 is expressed in all the cells. As the above totipotent cells, besides fertilized egg or early embryo, ES cells having multipotency can be specifically exemplified. Moreover, as for the method for introducing DNA into totipotent cells, well-known methods such as micro injection method, electric pulse method, liposome method, and calcium phosphate method can be used.

By transplanting nucleus of totipotent cells (cultured cells) of the above mouse into enucleat unfertilized egg and by initialization (nuclear transplantation), it is possible to introduce a large T-antigen gene of SV40 temperature-sensitive mutant tsA58 into ovum. Moreover, after transplanting a fertilizedegg, obtained from micro injection of a large T-antigen gene of SV40 temperature-sensitive mutant tsA58 into a female pronucleus of fertilized egg at the pronucleus stage, into the oviduct of tentative mother, a litter size is obtained, by selecting litter mates with the injected genes that are integrated in a stable manner consequently, congenic mice with a large T antigen genes of tsA58 integrated in chromosomes of cells of each organ, that is, transgenic mice, can be effectively produced at the time of the generation.

It has a characteristic to control the expression of differentiated trait inherent to the cell since immortalized natural killer cell of the present invention retains permanent proliferation ability at 33°C although the proliferation is suppressed at 37°C, and stopped at 39°C. Moreover, they are useful for researches on immune induction and modification, treatment for cancer, and viral disease, as well as for research on the development of diagnostic and therapeutic agents for diseases related to these pathologies at a cellular level since immortalized natural killer cell of the present invention shows satisfactory proliferation at 33°C even after 10 months of subculture, and retains the function as natural killer cells. Furthermore, since it retains the ability to attack and kill cancer cells and virus-infected cells without previous sensitization, it is not only useful as cell vaccine but also for screening of substances promoting or suppressing cellular cytotoxicity as shown below.

As for the method for the screen of substances promoting or suppressing cellular cytotoxicity of natural killer cells, there is no specific limitation as long as it is a method to culture the above immortalized natural killer cell line in the presence of a test substance, and to measure and estimate the level of cellular cytotoxicity of the immortalized natural killer cell line, that is, the level of the ability to kill a target cell without presensitization (Natural Killing) and/or a target cell coated with an antibody (ADCC), and it is generally performed by comparing with the measured level of controlled NK cell cultured in the absence of a test substance. Moreover, to use substances promoting or suppressing cellular cytotoxicity of natural killer cells obtained by the above screening method as immune induction and modification, therapeutic agent, agent for preventing and/or improving symptoms of various cancers and viral infections, and they are included in the present invention.

As for the cell vaccine of the present invention, it is not particularly limited as long as if the above immortalized natural killer cell line of the present invention is the major component. Yet, as the immortalized natural killer cell line mentioned above, human-derived immortalized natural killer cell line is preferable, and such immortalized natural killer cell line derived from human can be established by isolating natural killer cell from human peripheral blood, introducing a large T- antigen gene of SV40 temperature-sensitive mutant tsA58 , and repeating subculture; or by introducing a large T-antigen gene of SV40 temperature-sensitive mutant tsA58 into human embryonic stem cell (ES cell), differentiating this into natural killer cell in vitro, and repeating subculture. Furthermore, as for the immortalized natural killer cell line, it is preferable that those cells can proliferate at 33° C, but the proliferation is suppressed at 37° C. Cell vaccine of the present invention can act in vivo, ex vivo, or in vitro. For example, cell vaccine of the present invention, which is comprised of the suspension of immortalized natural killer cell line in the present invention, is injected into human body as therapeutic vaccine, and the safety may be assured since proliferation is suppressed at 37°C. Ordinarily, in order to enhance the safety of cell vaccine, heat treatment, radioactive treatment, treatment with C treatment or the like are necessary. However, it can be said that the immortalized natural killer cell line of the present invention is a vaccine with high potential of safety since such cell-inactivated treatment is not required, that is, these cells are able to proliferate at 33°C, whereas the proliferation is suppressed at 37°C .

Cell vaccine of the present invention, especially the cell vaccine whose major component is human-derived immortalized natural killer cell line, can be used advantageously for leukemia, various tumors, such as liver cancer, lung cancer, gastric cancer, and colon cancer, as well as infected diseases by various viruses, bacteria, and so on. The dosage of cell vaccine in the present invention cannot be determined in general, since it varies depending on the patient's age, weight, sex, type of cancer, its progression and symptom, or the like. However, it is possible to administer an equivalent amount of injected NK cells with the ongoing cell vaccine therapy to the patient. The cell vaccine of the present invention can be used for patient himself as well as for many other patients.

The present invention will be explained in detail with examples in the following, but the technical scope of the present invention is not limited to these examples.

### Example 1(The Generation of tansgenic mice)

Transgenic mice in which a large T-antigen gene of SV40 temperature-sensitive mutant tsA58 was introduced was generated in the following process.

### (Preparation of transgene)

For microinjection, modification of genomic DNA of SV40 temperature-sensitive mutant tsA58 by genetic engineering was used. Genomic DNA of tsA58 was open rung with restriction enzyme BamHI, introduced into BamHI site of pBR322, transferred SfiI sequence into SacII, and according to a common procedure, DNA for introduction was prepared from DNA clone pSVtsA58ori(-)-2 (Ohno T. et al., Cytotechnology, 165-172,1991) wherein origin of replication (ori) of SV40 isolated. In other words, pSVtsA58 ori(-)-2 of plasmid DNA obtained by amplifying large amount in E. coli, was digested with BamHI (Takara Shuzo Co., LTD.), separated by agarose gel electrophoresis (1% gel; Behringer), and after dissolving gel , phenol chloroform treatment and ethanol precipitation treatment were performed to collect DNA. The collected DNA was dissolved into TE buffer(10 mM Tris-HCl containing 1mM of EDTA; pH 7.6), and solution containing 170 µM/ml of purified DNA was obtained. The DNA solution was diluted with a buffer (10 mM of Tris-HCl containing 0.1 mM EDTA; pH 7.6) for injection, so that it becomes 5 µg/ml, to prepare DNA solution for injection. The prepared DNA solution was preserved at -20° C until injection operation.

### (Generation of A Transgenic Mouse)

Microinjection of DNA solution for injection use, which was prepared as above into mouse fertilized egg in pronucleus stage, was performed as follows. Eight-week old Wister mice, which were sexually matured, were bred in a light-dark cycle of 12 hours (4:00-16:00 as light hours) at a temperature of 23 ± 2 ° C in a humidity of 55±5%, and female estrous cycle was observed with vaginal smear and the day to perform hormone treatment was selected. First, 150 IU/kg pregnant mare serum gonadotropin (Nippon Zenyaku Kogyo Co., Ltd; PMS gonadotropin (PMSG)) was injected by intraperitoneally into a female mouse, and 48 hours later, 75 IU/kg human chorionic gonadotropin (Sankyo-Zoki Co.; Puberogen (hCG)) was administered to perform superovulation. Then, interbreeding by housing with a male was carried out. After 32 hours hCG adminstration, fertilized egg in pronucleus stage was collected by tubal perfusion. For tubal perfusion and culture of egg, mKRB solution (Toyoda Y. and Chang M. C., J. Reprod. Gertil., 36, 9-22, 1974) was used. The collected fertilized egg were treated with enzyme in mKRB solution containing 1 % hyaluronidase (Sigma) at 37°C for 5 minutes to remove cumulus cells, and washed three times with mKRB solution to remove enzyme. The resultant was stored in CO₂ incubator(in 5% of CO₂, 95% of Air, at 37 °C, saturating humidity) until DNA injection. The above-mentioned DNA solution was injected into female pronucleus of fertilizedmouse egg prepared in such a way. By transplanting the injected 228 eggs into nine tentative mothers for bearing, 80 littermates were obtained. The introduction of the injected DNA into mouse was assayed by PCR method of the DNA prepared from tail cut right after weaning. [primer used; tsA58-1A, 5' -TCCTAATGTGCAGTCAGGTG -3' (corresponds to site 1365-1384 : Seq. ID No.1), tsA58-1B, 5'-ATGACGAGCTTTGGCACTTG-3' (corresponds to locus of 1571-1590: Seq. ID No.2)]. As a result, 11 line of transgenic mice which survived until twelve-week old, when sexual maturation period is passed, was obtained from littermates of 20 mice (6 males, 8 females, 6 gender undetermined) admitted to be transgenic (male line: #07-2, #07-5, #09-6, #12-3, #19-5, female line: #09-7, #11-6, #12-5, #12-7, #18-5, #19-8). By interbreeding these transgenic mice of Go generation with wistar mice, transmission of genes over the next generation was confirmed in either 2 lines of male founder (#07-2, #07-5) and 3 lines of female founder (#09-7, #11-06, #19-8).

### Example 2 (Isolation and Preparation of NK cells from Spleen)

The isolation and preparation of natural killer cell from spleen were carried out as follows. Spleen was extracted from a transgenic mouse wherein a large T-antigen gene of SV40 temperature-sensitive mutant tsA58, obtained in Example 1, was introduced, and NK cells were isolated by NK 1.1+MACS (magnetic beads method). The cells were cultured in a medium,[RPMI-1640(SIGMA, 16-700-49), 10%(v/v) FBS (SIGMA, Lot:40k2368) 50 µM 2 penicillin, 50 µg/mL streptomycin, (Dainippon Pharmaceutical Co., Ltd. 16-700-49), 50 µM 2-merucaptoethanol, 1 mM pyruvic acid, 1 x MEM Non-essential Amino Acid Solution (GIBCO BRL Co. 11140-850), containing 1000 U/mL interleukin-2,(Pepro Tech, 200-02 or Strathmann Biotech, IL2-50)], a cytokine promoting proliferation of NK cells, for approximately one month. The resultant was diluted to 0.2 per well, and inoculated on a 96 well-plate. These cells were cultured in an incubator at 33° C in 5% CO₂. Among 244 wells, proliferation of cell was observed from around 30 wells, and 8 lines that showed considerable proliferation, i.e. , TNK1, TNK2, TNK3, TNK4, TNK6, TNK8, TNK9, and TNK10 were selected. For these eight immortalized natural killer cell lines, it was examined whether they normally retained the following characteristics of natural killer cells.
1. Kill virus infected or cancer cells without presensitization.
2. Kill cells coated with an antibody.
3. Produce cytokines such as IFN-γ in response to stimulation.
4. Have characteristic cell surface antigen (NK1.1 etc.).
5. Proliferate and activate with interleukin-2.

### Example 3 (Morphologic Observation by Wright-Giemsa Staining Method)

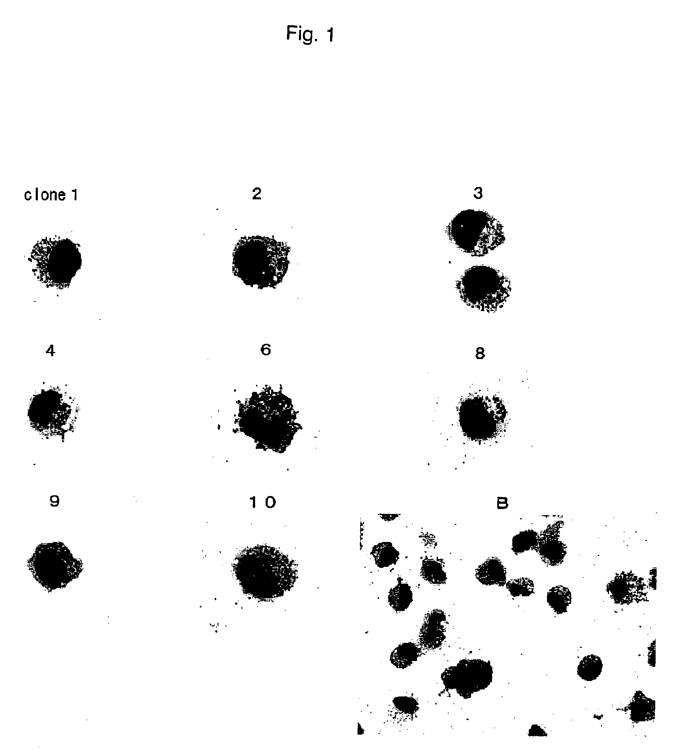

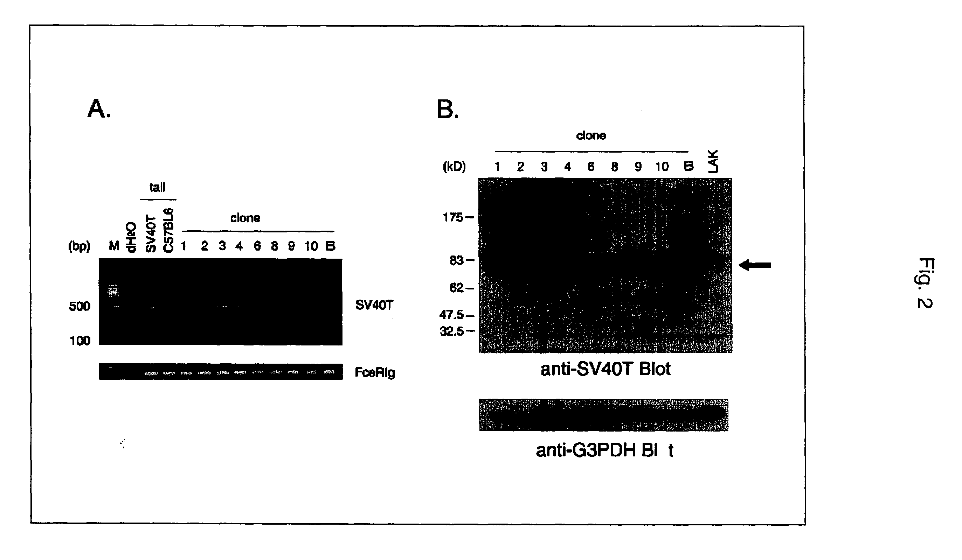

Natural killer cells have granules related to killing target cells, and the granules can be visualized by Wright-Giemsa staining method. The eight immortalized natural killer cell lines and immortalized natural killer cell lines before dilution Example 2 (TNKb) were adhered with Cytospin on a slide glass, stained by Wright-Giemsa Method (Wright Staining Solution, Giemsa Staining Solution, both from Merk Ltd.) and visualized. The results are shown in Fig. 1. In Fig. 1, B shows the above TNKb. As a result, each immortalized natural killer cell was stained in purple, and azurophilic granules were confirmed. From this result, each immortalized natural killer cell was confirmed to have characteristics of NK cells, being morphologically normal.

### Example 4 (Confirmation to be derived from SV40 Transgenic Mouse)

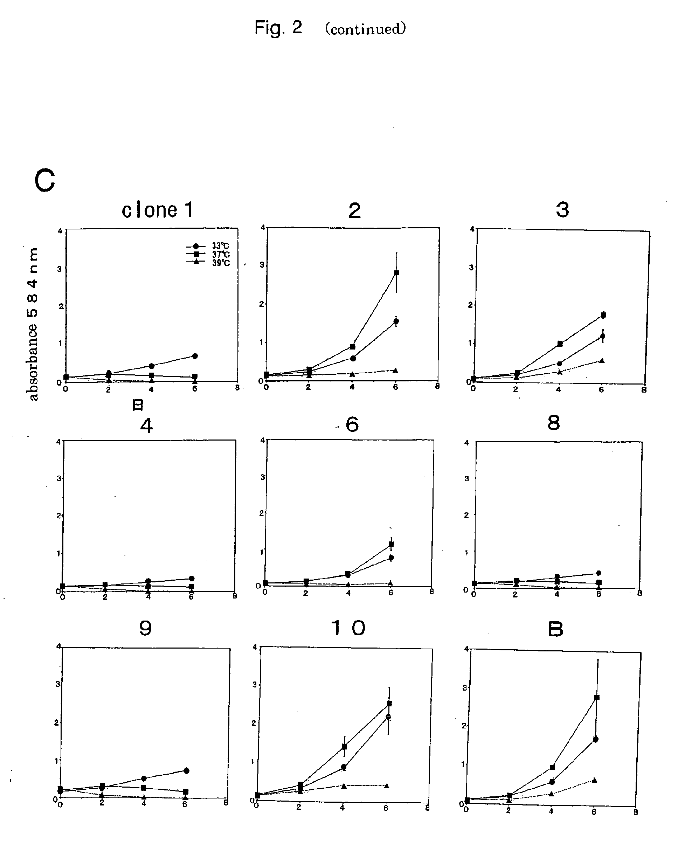

For the eight obtained immortalized natural killer cell lines, TNKb and so on, the presence of SV40 T genes was confirmed by PCR method. [Primer used; SV404441S, 5'-GGAGGAGTAGAATGTTGAG -3': Seq. ID. No.3, SV40T 4892AS, 5'-GTGTTGATGCAATGTACTGC=3'. Seq. ID. No.4]. FCε Rig was used as a control. As a result, characteristic band of SV40 gene was detected for the eight immortalized natural killer cell lines, and the presence of transgene was confirmed. In the same way, SV40T protein was detected by western blot method. As a result, characteristic band of SV40 T protein was detected for the eight immortalized natural killer cell lines, and the presence of the protein was confirmed. G3PDH was used as a control. Moreover, due to the construction of the transgene, cell line derived from this mouse is immortalized at 33°C, while immortalization is canceled, proliferation is stopped, and perishes at 39°C. MTT (3-(4,5-dimethylthizaol-2-yl)-2, 5-diphenyl tetrazo-lium bromide) is cleaved by dehydrogenase of mitochondrion intima and so on, and produces reddish violet MTT-formazan. Through examining proliferation of the eight immortalized natural killer cell lines by MTT assay based on the fact that this color reaction is proportional to the proliferation ability of cells, the above temperature-dependent proliferation was observed in each of natural killer cell line. From these facts, each of eight immortalized natural killer cell lines were confirmed to be a cell line derived from SV40T transgenic mouse. The results are shown in Figure 2.

### Example 5 (Request of a Proliferation Factor)

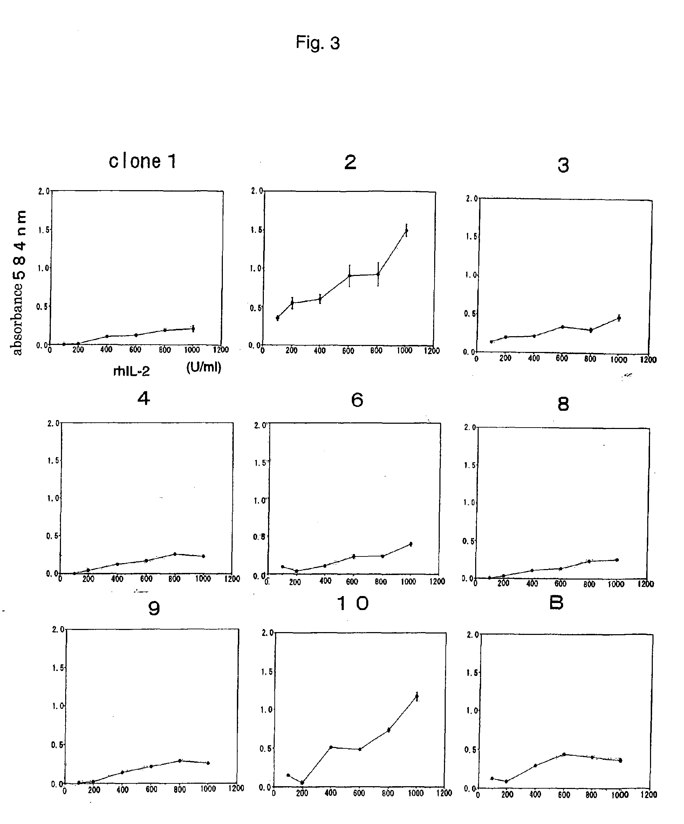

There are many immunocytes which used to be cultured in a medium including a particular proliferation factor, when they are isolated ex vivo. Natural killer cells are known to require interleukin-2 for proliferation. As proliferation factor is valuable and precaution is necessary for long-term preservation, it is most preferable if proliferate is possible without this. The cell line of the present invention has been cultured in a medium including 1000 U/mL of interleukin-2 from the initiation of the culture, to investigate whether it is possible or not to proliferate without it or by decreasing the amount. After culturing for six days with 1000 U/mL of human recombinant interleukin-2 (Pepro Tech or Strathmann Biotech), proliferation of natural killer cells was examined by MTT assay. The results are shown in Fig. 3. As a result, for any of these eight immortalized natural killer cell lines and TNKb, proliferation was found to be the best at 1000 U/mL. From the results of this experiment, since the obtained natural killer cell lines proliferate in a interleukin-2 concentration-dependent manner, it has been clarified that it is better not to decrease interleukin-2.

### Example 6 (Cellular Cytotoxicity)

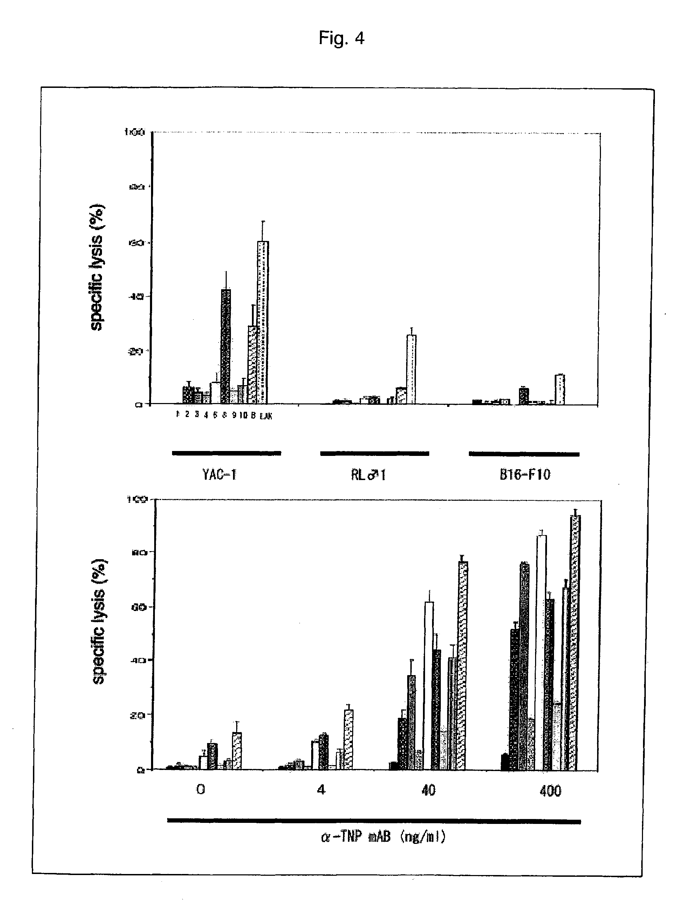

Natural killer cells have two killing mechanisms. One is that of killing infected cells or cancer cells (Natural Killing) without presensitization, while the other is that of killing cells coated with an antibody (ADCC). It was investigated by ⁵¹Cr releasing test whether the eight natural killer cell lines and TNKb (E) retain these two killing mechanisms or not. The Natural Killing Ability was measured by using YAC-1 cells sensitive to cytotoxicity by natural killer cells, RL♂ 1 cell, and B16-F10 cell for cells as Target cells (T), with the proportion of E : T=10 : 1. Moreover, ADCC ability was measured by using EL-4 cells, with Trinitrophenol (TNP) bound to the cell surface, together with anti-mouse TNP IgG1 antigen immune complex, as targeting cells (T), with the proportion of E:T=10:1. The results are shown in Fig. 4. In Fig. 4, LAK signifies fresh natural killer cells derived from spleen stimulated and proliferated for seven days in a medium including 1000 U/mL interleukin-2. From Fig. 4 (above), since TNK 8 line showed a particularly high Natural Killing ability as LAK, it has been clarified that the TNK 8 line was useful especially for clarification of Natural Killing mechanism. Moreover, from Fig. 4 (below), in the case of immune complex with antibody, even for EL-4 cells which are not sensitive to cell cytotoxicity by natural killer cells, each of the eight immortalized natural killer cell line, especially TNK2, TNK3, TNK6, TNK8 , and TNK10, showed a high antibody concentration-dependent ADCC ability.

### Example 7 (Cell Surface Antigen)

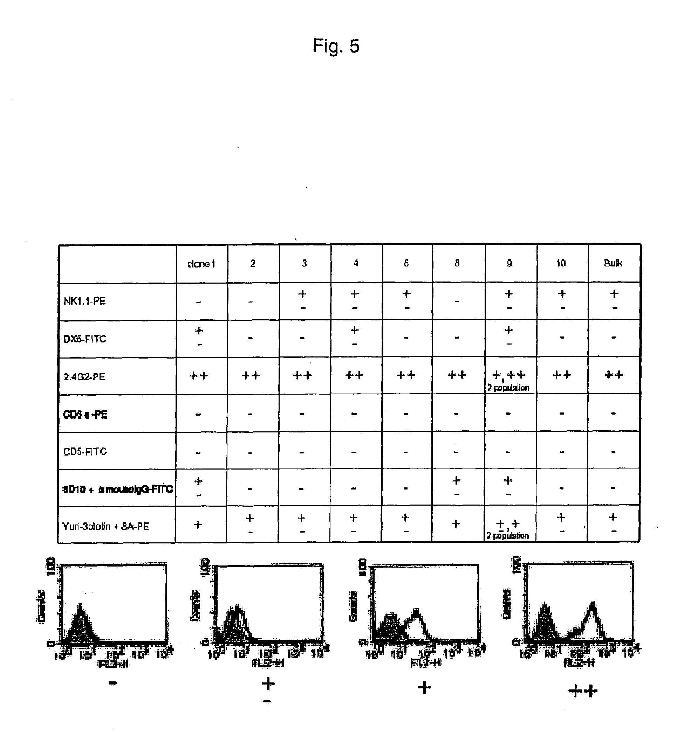

Natural killer cells express their surface characteristic antigens such as NK1.1, DX5, FcγRIII, Ly49H, CD94, and so on, which are shown in Fig. 5. It was confirmed by flow cytometry whether the eight immortalized natural killer cell lines and TNKb retain this antigen expression normally. Cell suspension was produced, and specific monoclonal antibodies were reacted on ice for 15 minutes. After washing a buffer solution, those requiring secondary reaction, were reacted for 15 minutes on ice with labeled secondary antibodies. Cells labeled with monoclonal antibodies was analyzed with FACScalibur (Becton Dickinson, Flow Cytometry analyzing device). The results are shown in Fig. 5. From Fig. 5, it has been clarified that expression of several characteristic antigens was lost or decreased in the eight of immortalized natural killer cell lines and TNKb.

### Example 8 (cytokine production ability)

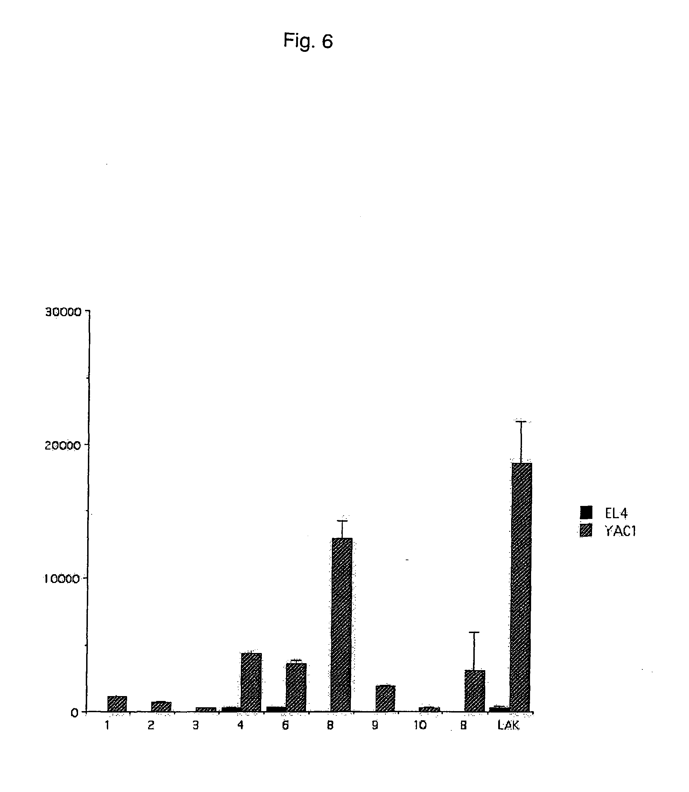

Natural killer cells produce cytokines such as IFN-γ in response to stimulation. It was examined whether the eight natural killer cell lines and TNKb (E) produce IFN-γ in response to stimulation by contact with target cells (T). By using as target cells, YAC-1 sensitive to NK cells, or EL-4 non-sensitive to NK cells coculture was performed in a contacted situation within 96-well culture plate. After 24 hours, conditioned culture supernatant was collected, and IFN-γ was measured by ELISA method. The results are shown in Fig. 6. As a result, the eight immortalized natural killer cell lines and TNKb did not produce cytokine in contact with the unimpaired EL- 4 , whereas in contact with YAC-1, they produced cytokine and it was confirmed to retain normal function. Moreover, since eight TNK lines showed a particularly high cytokine production ability in the same manner as LAK, it has been clarified that eight TNK lines were especially useful for elucidation of the cytokine production mechanism.

### Industrial Applicability

According to the present invention, it is possible to obtain immortalized natural killer cell line which continues to proliferate stably, and by using the cell line, it is possible to carry out advantageously researches on target recognition mechanism of natural killer cells, other detailed researches on natural killer cells, and research related to treatment for cancer and for viral infection with natural killer cells. Moreover, according to the present invention, since it retains the functions and characteristics of the natural killer cells, it is possible to perform screening of useful substance promoting or suppressing the action of NK cells.

## Claims

1. An immortalized natural killer cell line derived from spleen cells that proliferates and activates in the presence of Interleukin-2, has azurophilic granules within cytoplasm, and retains an ability to kill a target cell without presensitization and/or an ability to kill a target cells coated with an antibody.

2. The immortalized natural killer cell line, according to Claim 1, that can proliferate at 33°C, while the proliferation is suppressed at 37°C.

3. The immortalized natural killer cell line according to Claim 1 or 2, wherein the cell line is derived from a rodent.

4. The immortalized natural killer cell line according to Claim 3, wherein the rodent is a mouse.

5. The immortalized natural killer cell line according to Claim 4, wherein the immortalized natural killer cell line has DX5, FcγRIII, Ly49H and CD94 as cell surface antigens, whereas it does not have NK1.1.

6. The immortalized natural killer cell line according to Claim 4, wherein the immortalized natural killer cell line has FcγRIII and CD94 as cell surface antigens, whereas it does not have NK1.1, DX5, and Ly49H.

7. The immortalized natural killer cell line according to Claim 4, wherein the immortalized natural killer cell line has NK1.1, FcγRIII, and CD94 as cell surface antigen, whereas it does not have DX5 and Ly49H.

8. The immortalized natural killer cell line according to Claim 4, wherein the immortalized natural killer cell line has NK1.1, DX5 , FcγRIII and CD94 as cell surface antigens, whereas it does not have Ly49H.

9. The immortalized natural killer cell line according to Claim 4, wherein the immortalized natural killer cell line has FcγRIII, Ly49H, and CD94 as cell surface antigens, whereas it does not have NK1.1 and DX5.

10. The immortalized natural killer cell line according to Claim 4 , wherein the immortalized natural killer cell line has NK1.1, DX5, FcγRIII, Ly49H, and CD94 as cell surface antigens.

11. Immortalized natural killer cell line TNK1 (FERM BP-08518).

12. Immortalized natural killer cell line TNK2 (FERM BP-08519).

13. Immortalized natural killer cell line TNK3 (FERM BP-08520).

14. Immortalized natural killer cell line TNK4 (FERM BP-08521).

15. Immortalized natural killer cell line TNK6 (FERM BP-08522).

16. Immortalized natural killer cell line TNK8 (FERM BP-08523).

17. Immortalized natural killer cell line TNK9 (FERM BP-08524).

18. Immortalized natural killer cell line TNK10 (FERMBP-08525).

19. Immortalized natural killer cell line TNKb (FERM BP-08526).

20. A method for producing immortalized natural killer cell line, by culturing natural killer cells obtained from spleen of a transgenic mouse, in which a large T-antigen gene of SV40 temperature-sensitive mutant tsA58 is introduced, and by establishing a cell line that proliferates and activates in the presence of interleukin-2, having azurophilic granules within cytoplasm, and retaining an ability to kill a target cell without presensitization, and/or an ability to kill a target cell coated with an antibody.

21. A method for screening substances promoting or suppressing cellular cytotoxicity of natural killer cells, wherein the immortalized natural killer cells according to any one of Claims 1 to 19 are cultured in the presence of a test substance, and the cellular cytotoxicity level of the immortalized natural killer cells is measured/estimated.

22. A substance promoting or suppressing cellular cytotoxicity of the natural killer cells obtained by the screening method according to Claim 21.

23. A cell vaccine having the immortalized natural killer cells according to any one of Claims 1 to 19 as major components.

24. The cell vaccine according to Claim 23, wherein the immortalized natural killer cell line can proliferate at 33° C while the proliferation is suppressed at 37°C.


SEQUENCE LISTING

<110> JAPAN SCIENCE AND TECHNOLOGY AGENCY

<120> Immortalized natural killer cell strain

<130> P029P01PCT

<140>
<141>

<150> JP P2002-316870
<151> 2002-10-30

<160> 4

<170> PatentIn Ver. 2.1

<210> 1
<211> 20
<212> DNA
<213> Artificial Sequence

<220>
<223> Description of Artificial Sequence:tsA58-1A

<400> 1

tcctaatgtg cagtcaggtg                                                    20

<210> 2

<211> 20

<212> DNA

<213> Artificial Sequence

<220>

<223> Description of Artificial Sequence:tsA58-1B

<400> 2

atgacgagct ttggcacttg                                                    20

<210> 3

<211> 19

<212> DNA

<213> Artificial Sequence

<220>

<223> Description of Artificial Sequence:SV404441S

<400> 3

ggaggagtag aatgttgag                                                     19

<210> 4

<211> 20

<212> DNA

<213> Artificial Sequence

<220>

<223> Description of Artificial Sequence:SV40T4892AS

<400> 4

gtgttgatgc aatgtactgc 20